# EUROPEAN PATENT APPLICATION

(11) **EP 4 389 108 A2**
(43) Date of publication of application: **26.06.2024**
(21) Application number: 23214928.6
(22) Date of filing: 07.12.2023
(51) Int. Cl.: A61K 8/19, A61K 8/9789, A61K 8/9794, A61Q 5/06

(54) **HAIR COSMETIC**

(30) Priority: 22.12.2022 JP 2022205434
(71) Applicant: DIC CORPORATION, Itabashi-ku Tokyo 174-8520 (JP)
(72) Inventor: SEKIKAWA, Hiroshi, Kamisu-shi, 314-0193 (JP); TANAKA, Yuka, Sakura-shi, 285-8668 (JP)
(74) Representative: TBK

(57) **Abstract**

A hair cosmetic containing a water-insoluble pigment composite including a complex of a natural pigment and a substrate is unlikely to cause pigmentation; the pigment does not penetrate to the inside of the hair because of the insolubility of the pigment composite in water. The water-insoluble pigment composite according to the present invention, furthermore, exhibits good color strength owing to the concealing effect of the substrate even when used on black hair. Based on the finding that water resistance, color strength, and washability are substantially improved, the present invention was completed.

## Description

### BACKGROUND OF THE INVENTION

### 1. Field of the Invention

The present invention relates to a hair cosmetic.

### 2. Description of the Related Art

In the related art, hair cosmetics (also known as hair dyes, hair bleaches, hair manicures, color rinses, hair chalks, hair mascaras, color sprays, hair gels, hair oils, etc.) are required to have water resistance, color strength, and washability after application to the hair.

It is described that a known category of hair cosmetics derived from natural pigments is temporary hair dyes having the ability to provide cumulative hair dyeing, and they are capable of dyeing hair through repeated use (Japanese Unexamined Patent Application Publication No. 2004-250394). A hair dye made with a safe pigment and a method for using it, furthermore, have been proposed because of concerns about the carcinogenicity of and allergies to known acid dyes and coupling agents used in hair dyeing (Japanese Unexamined Patent Application Publication No. 2012-116829) .

These hair dyes described in the literature, however, exhibit poor water resistance, low color strength, and limited washability because of the nature of being derived from natural pigments.

### SUMMARY OF THE INVENTION

Accordingly, it is an object of the present invention to provide a hair cosmetic improved in terms of water resistance, color strength, and washability, which have been disadvantages of natural pigments.

After extensive research to achieve the object, the inventors found that a hair cosmetic containing a water-insoluble pigment composite including a complex of a natural pigment and a substrate exhibits substantially improved water resistance, color strength, and washability. Based on this finding, the inventors completed the present invention.

That is, the present invention includes the following aspects.
[1] A hair cosmetic containing a water-insoluble pigment composite including a complex of at least one natural pigment and at least one substrate.
[2] The hair cosmetic according to 1, wherein a ratio between an amount of the natural pigment and an amount of the substrate in the water-insoluble pigment composite is the natural pigment:the substrate = 0.1:99.9 to 90:10 as a ratio by mass.
[3] The hair cosmetic according to 1 or 2, wherein the substrate in the water-insoluble pigment composite is at least one selected from a metal oxide, a metal hydroxide, a clay mineral, hydroxyapatite, or an organic polysaccharide.
[4] The hair cosmetic according to any one of 1 to 3, wherein the natural pigment in the water-insoluble pigment composite is at least one selected from a flavonoid pigment, a carotenoid pigment, a porphyrin pigment, or a chromoprotein.
[5] The hair cosmetic according to any one of 1 to 3, wherein the natural pigment in the water-insoluble pigment composite is at least one selected from annatto extract, chlorophyll pigment, Carthamus yellow, or phycocyanin pigment.

According to the present invention, there can be provided a hair cosmetic that exhibits high water resistance and high color strength and is unlikely to cause pigmentation. The water-insoluble pigment composite according to the present invention does not dissolve in water. By virtue of this, the pigment does not penetrate to the inside of the hair, and pigmentation is unlikely to occur. The water-insoluble pigment composite according to the present invention, furthermore, exhibits good color strength owing to the concealing effect of the substrate even when used on black hair.

### DESCRIPTION OF THE PREFERRED EMBODIMENTS

The water-insoluble pigment composite according to the present invention will now be described in detail. The following description of requirements is an example in an aspect of the present invention and is not intended to be limiting.

### Natural pigment

A natural pigment used in the present invention can be any pigment of natural origin, such as the red cabbage color, the red radish color, the annatto extract, the sepia (squid ink) color, the crucumin pigment, the cacao color, carotene pigments, carotenoid pigments, Gardenia red, Gardenia blue, Gardenia yellow, the chlorophyll pigment, the phycocyanin pigment, the kaoliang color, the cochineal extract, the saffron color, the beefsteak plant (*shiso*) color, sandal wood red, the spirulina color, the onion color, the tamarind color, the butterfly pea color, the paprika color, the tomato color, the hibiscus color, beet red, the grape skin extract, the Haematococcus color, the Monascus color, Carthamus red, Carthamus yellow, berry colors, the marigold color, the purple sweet potato color, the purple corn color, the purple yam color, caramel pigments, and vegetable carbon black. Biosynthesized, enzymatically synthesized, or chemically synthesized forms of these pigments can also be used.

Of these, examples of pigments particularly suitable for use in the present invention include carotenoid pigments, porphyrin pigments, flavonoid pigments, and chromoproteins.

For carotenoid pigments, pigments such as red pigments extracted from carrots, tomatoes, and paprikas and yellow pigments extracted from citrus fruits, gardenias, annattos, and marigolds are known. Green leafy vegetables are also rich in carotenoid pigments.

Carotenoids are substances that belong to the class of terpene compounds, particularly to the subclass of tetraterpenes. Terpenes are important compounds for plants, with more than 20,000 compounds known. For carotenoid pigments, pigments such as hydrocarbon pigments, including β-carotene and lycopene, xanthophyll pigments, including astaxanthin, capsanthin, and lutein, as well as bixin, norbixin, and crocin are known.

Porphyrin pigments have four pyrrole rings, and their metal complexes, contained in substances such as chlorophyll, which serves the roles of light absorption and photoelectron transfer in photosynthesis, and heme in hemoglobin, which transports oxygen in blood, are compounds that take an important role in living organisms. Porphyrin-metal complexes are used in many fields as photoelectronic functional materials, metal complex catalysts, and molecular conductive materials and are known to exhibit a really wide variety of functionalities with different substituents around the porphyrin, different central metals, and different ligands in the axial positions.

Flavonoids constitute one major category of polyphenols. "Flavonoid" is a generic term for substances having a specific chemical structure, and they are contained in sources such as leaves, stems, and trunks of plants. Flavonoids are substances that plants produce to protect themselves, for example from ultraviolet radiation and pests, and serve as the basis for pigments and bitter-tasting food ingredients. Flavonoids, furthermore, are classified into flavonols, flavones, catechins, flavanones, anthocyanins, isoflavones, etc., according to their structure.

Examples of flavonoid pigments include pigments such as flavonols, anthocyanin pigments, which produce various color tones, and chalcone and aurone, which produce deep yellow colors.

### Chromoproteins

"Chromoprotein" is a generic term for proteins that are in the form of a complex with a pigment in their natural state. Chromoproteins are present in animal and vegetable cells, animal body fluids, and vegetable sap and produce various color tones and physiological functions by virtue of a prosthetic group containing a pigment. Hemeproteins are proteins containing an iron-porphyrin complex salt bound to them, and the ratio of binding between the protein and heme is 1:1, 1:2, 1:4, etc. Hemoproteins are widespread in nature, and examples include hemoglobin, myoglobin, cytochromes, catalase, and peroxidases.

Metal complex compounds are proteins containing a metal complex ion bound to them, and examples include copper proteins, such as hemocyanin, and iron proteins, such as ferritin. Ferritin is present in anatomical structures such as the spleen, the small intestinal mucosa, and the liver and is believed to be involved in the absorption of iron during iron storage and digestion in the body of living organisms.

Phycochromoproteins are proteins containing a pyrrole derivative bound to them, and examples include phycoerythrin, which produces the red color of red algae, and phycocyanin, which produces the indigo color of blue-green algae. Phycochromoproteins are contained in chloroplasts together with chlorophyll and carotenoids and are believed to be accessory pigments in photosynthesis.

Flavoproteins have flavin mononucleotide or flavin adenine dinucleotide as their prosthetic group. Since all of them have functions as oxidoreductases, flavoproteins are also referred to as flavoenzymes. Examples include amino acid oxidases and xanthine oxidase.

Carotenoid proteins are proteins containing a carotenoid bound to them. Examples include proteins containing vitamin A bound to them, and rhodopsin is one of such proteins.

### Phycocyanin

For use as a chromoprotein in the present invention, phycocyanin is the most preferred because it produces a vivid blue color. Phycocyanin is a chromoprotein and has phycocyanobilin as its chromophore. Phycocyanin has a structure in which phycocyanobilin and a protein are bound together.

Examples of phycocyanins according to the present invention include phycocyanins of algae origin, such as phycocyanin from blue-green algae, phycocyanin from red algae, and phycocyanin from cryptophytes. Of these, phycocyanin from blue-green algae is particularly preferred because it can be collected abundantly.

Examples of blue-green algae include blue-green algae in genera such as *Spirulina, Arthrospira, Aphanizomenon, Fischerella, Anabaena, Nostoc, Synechocystis, Synechococcus, Tolypothrix, Aphanothece, Mastigocladus,* and *Pleurocapsa.* Of these, blue-green algae in the genera *Spirulina* and *Arthrospira,* which are produced in industrial scales and have proven their safety, are particularly preferred, and blue-green algae in the genus *Spirulina* are more preferred.

The source material for preparing the phycocyanin, furthermore, can be fresh blue-green algae or may be dried blue-green algae. The dried blue-green algae may be a dry form of the algae prepared by drying fresh blue-green algae following the standard method for it or can be commercially available dry algae.

Phycocyanin can be obtained by, for example, suspending blue-green algae in water or a buffer, such as a phosphate buffer or citrate buffer, and extracting phycocyanin in the blue-green algae.

For the method for extracting phycocyanin, there is no particular limitation; commonly known methods can be used.

An example of a preferred form of an extraction method is the extraction method described in Japanese Unexamined Patent Application Publication No. 2006-230272. A specific example is the extraction method described in Extraction Method (i) below. With such extraction method (i), high-purity phycocyanin with a vivid color tone can be obtained.

### Extraction Method (i)

Extraction method (i) includes:
a first step, in which an extract is obtained in which phycocyanin in blue-green algae has been extracted in an aqueous suspension;
a second step, in which a calcium salt and a phosphate are allowed to react together in the extract to produce calcium phosphate while impurities in the phycocyanin are adsorbed onto the calcium phosphate to give a saturated adsorbent; and
a third step, in which blue-green algae residue and the saturated adsorbent are removed from the extract.

It is more preferred that extraction method (i) above be extraction method (ii) below.

### Extraction Method (ii)

Extraction method (ii) includes:
a first step, in which an extract is obtained in which phycocyanin in blue-green algae has been extracted in an aqueous suspension;
a second step, in which a calcium salt and a phosphate are allowed to react together in the extract to produce calcium phosphate while impurities in the phycocyanin are adsorbed onto the calcium phosphate to give a saturated adsorbent;
a third step, in which blue-green algae residue and the saturated adsorbent are removed from the extract; and
a step of adding a chelating agent to the extract before the third step.

The phycocyanin used in the present invention was LINABLUE G1 (manufactured by DIC LIFETEC Co., Ltd., 55% trehalose, 40% spirulina extract, and 5% trisodium citrate), a commercially available mixture of phycocyanin with a stabilizing agent. As described in Japanese Unexamined Patent Application Publication No. 11-299450, the trehalose is used to improve thermostability, and the citrate is used as a pH-adjusting agent. It should be noted that the phycocyanin pigment is contained as the base component within the spirulina extract.

In the present invention, one natural pigment may be used alone, or multiple natural pigments may be used together. Depending on the application, a premix of natural pigments may be prepared to the desired hue and combined with the substrate, or natural pigments may be individually combined with a substrate to give complexes, and then these complexes, or water-insoluble pigment composites, may be mixed together.

### Metal or Metal Compound

A metal or metal compound used in the present invention can be any metal or compound as long as it has a supporting function for insolubilizing the natural pigment. Examples include pure metals, metal oxides, and metal hydroxides, and aluminum, aluminum oxides, and aluminum hydroxides are particularly suitable for use. For a metal or the metal element in a metal compound used in the present invention, examples include the metals belonging to groups 1 to 15 in the Periodic Table of Elements excluding those in periods 1 and 2. Of these, metal elements such as iron, cobalt, nickel, zinc, aluminum, and titanium in particular can be used, and one or more selected from such metal elements can be used. In particular, a metal or metal compound not harmful to the human body in food or cosmetic applications is preferred, and aluminum, titanium, and zinc are particularly preferred in an embodiment of the present invention. In an embodiment of the present invention, it is preferred that the metal or metal compound be a hydroxide or oxide of a metal so that the physical adsorption of the natural pigment on the metal or metal compound will be further strengthened, and aluminum hydroxide, titanium oxide, and zinc oxide are particularly suitable for use. It is more preferred to use a slurry, for example of aluminum hydroxide, titanium oxide, or zinc hydroxide, obtained by adding an alkali to a chloride, such as aluminum chloride, titanium tetrachloride, or zinc chloride, because this makes the physical adsorption of the natural pigment even stronger. It is, furthermore, more preferred that the surface of the metal or metal oxide be covered with the natural pigment for further strengthened physical adsorption.

### Hydroxyapatite

Hydroxyapatite, used in the present invention, is a type of calcium phosphate and is the base component of teeth and bones. Present in the natural world as a constituent element of minerals and living organisms, hydroxyapatite is commonly used in medical devices and dental materials (e.g., an implant coating, an osteoplastic material, and artificial dental roots) as their constituent element because of its high biocompatibility. Hydroxyapatite, furthermore, has its unique nature: it maintains its crystal structure even after a subset of ions in the crystal is replaced. By virtue of this, various functions, such as the function of selectively adsorbing a protein and a catalytic function, can be imparted to hydroxyapatite by fine-tuning the ions in the crystal or the shape of the crystal, making hydroxyapatite a substance commonly used in the field of chemical industry as well.

Hydroxyapatite has a greater specific surface area and higher adsorption potential with decreasing crystallinity. In this respect, hydroxyapatite used as a support in the present invention is preferably low-crystallinity hydroxyapatite having a Ca/P (molar ratio) of 1.4 to 1.8. Likewise, for adsorption capacity reasons, the hydroxyapatite is preferably of a type whose specific surface area measured by nitrogen gas adsorption is 10 m²/g or more (more preferably 40 m²/g or more). Since the adsorption capacity tends to increase with increasing bulk volume of the hydroxyapatite, the hydroxyapatite is preferably of a type having a bulk volume of 500 mL/100 g or more (more preferably 900 mL/100 g or more). The average particle size is preferably from 0.1 to 40 um, more preferably from 10 to 30 µm.

### Clay Mineral

Clay minerals are minerals that constitute clay, and their base component is a sheet silicate mineral (phyllosilicate mineral). Sheets of metal ions (aluminum, sodium, calcium, etc.) and silicic acid bonded together are formed in layers. By virtue of their ability to easily take water, metal ions, and, in some cases, even organic substances into the gaps between these sheets and release them, clay minerals are used as functional materials, for example for humidity control, ion exchange, and catalysis, in household goods and many more fields. Clay minerals are also used as ceramic materials and raw materials for pottery.

Examples of clay minerals used in the present invention include bentonite, hectorite, smectite, kaolinite, montmorillonite, sericite, illite, glauconite, chlorite, talc, and zeolite.

Of these, bentonite, hectorite, and smectite are particularly desirable for use as clay minerals in the present invention because of their high compatibility with pigments, high insolubilizing potential, and ability to allow the pigment to achieve superior color strength.

The crystal phase of bentonite, hectorite, and smectite bears a negative charge. Between the layers of its sheet-shaped structure, the crystal phase holds positive ions that correct the charge imbalance. By virtue of the broad expanse of the layer surfaces in relation to the thickness of the sheets, these clay minerals exhibit high compatibility with pigments.

"Organophilization" refers to a process of replacing metal cations contained in a clay mineral with organic ions having hydrophobicity. Organic bentonite, organic hectorite, and organic smectite, which have undergone organophilization, each have a hydrophobic surface that exhibits strong hydrophobic interactions with the hydrophobic moiety that natural pigments, such as the annatto extract, the chlorophyll pigment, Carthamus yellow, and the phycocyanin pigment, have within their chemical structure. Through organophilization, therefore, clay minerals become more likely to adsorb pigments. For easier pigment adsorption and for adsorption capacity reasons, it is preferred in the present invention to use an organophilized clay mineral.

As for the shape of the particles of the clay mineral used, it is preferred that the secondary particle size be from 0.1 to 100 µm, more preferably from 5 to 50 um. Water-insoluble Pigment Composite

Many of natural pigments used in the present invention are of natural origin. Basically, therefore, they are in the form of dye and thus are water-soluble. In the present invention, however, the inventors have found that a natural pigment forms a firm complex with a substrate through the modes of action of coating, impregnation, and adsorption and, as a result, becomes insoluble in water by forming a water-insoluble pigment composite. The formation of a complex in this context is not limited to modes of action such as coating, impregnation, and adsorption; the inventors define it as a process in which the natural pigment acquires insolubility in water by interacting with hydroxyapatite or a clay mineral through physical or chemical adsorption, rather than simply forming a mixture.

The water-insoluble pigment composite used in the present invention can be used with the ratio between the amount of the natural pigment and that of the substrate set to the natural pigment:the substrate = 0.1:99.9 to 90:10 as a ratio by mass. Preferably, the natural pigment:the substrate = 1:99 to 80:20.

### Method for Producing the Water-Insoluble Pigment Composite

As for the method for producing the water-insoluble pigment composite according to the present invention, the method of mixing the natural pigment and the substrate together in a solvent is preferred because it allows the most uniform water-insoluble pigment composite to be produced.

In a method for producing the water-insoluble pigment composite in which the materials are mixed together in a solvent, 1) a dispersion of the substrate is prepared first. 2) Separately, the natural pigment is dissolved in water, and thereby an aqueous solution is prepared. 3) Then the two liquids are mixed together, and thereby a water-insoluble pigment composite is prepared. Step 3) may be performed by mixing the aqueous solution of the natural pigment into the dispersion of the substrate or may be by the opposite, i.e., mixing the dispersion of the substrate into the aqueous solution of the natural pigment. Alternatively, the pigment composite may be prepared by mixing the two liquids together in small portions. As for the mixing temperature, the liquids may be mixed together at room temperature or while being heated. It is preferred to mix them together at 10°C to 60°C, more preferably at 20°C to 50°C, considering the decomposition temperature of the natural pigment alone. 4) The pH of the mixture is adjusted to cause the natural pigment and the substrate to form a complex as a water-insoluble pigment composite.

A water-insoluble pigment composite can be obtained by filtering the resulting mixture and drying the residue. While the mixture is being filtered, for example using a Nutsche filter, the formation of a complex by the natural pigment and the substrate can be confirmed by observing that the filtrate is not colored. The wet cake of the water-insoluble pigment composite, furthermore, is repeatedly washed with water more times. The filtrate remains likewise colorless and transparent, and it can be confirmed from this that the pigment component has not flown out. The resulting water-containing wet cake of the water-insoluble pigment composite can be dried, for example at room temperature, by heating, in a vacuum, or by drying under reduced pressure, to give a dry water-insoluble pigment composite. The drying method and the dryer are not limited; they can be of any type as long as they are an ordinary method and ordinary equipment.

The water-insoluble pigment composite according to the present invention can be used either as the wet cake, which contains water, or as the dry water-insoluble pigment composite, which has been dried; it can be used in different forms according to the purpose of use. When it is used in a water-based dispersion or ink, the wet cake can be used directly. When the pigment composite is used in a solvent-based dispersion, the wet cake can be used after the replacement of water with the solvent. The dry water-insoluble pigment composite can be used directly and, naturally, can also be used after being dispersed again, for example in water, an organic solvent, or a resin solution. Stabilizing Agent and Additives

Naturally, other organic pigments, inorganic pigments, dyes, and coloring substances can be mixed into the water-insoluble pigment composite according to the present invention to any percentages. This allows the desired and required hue to be obtained. It is also possible to add a stabilizing agent and additives to the water-insoluble pigment composite according to the present invention to further impart light fastness and heat resistance to the pigment composite.

The stabilizing agent and additives can be added to one of the aqueous solution of the metal hydroxide or the aqueous solution of the natural pigment or both or may be added to the prepared water-insoluble pigment composite. Oily Base

The hair cosmetic according to the present invention, particularly when used as a hairdressing material or cosmetic, preferably contains an oily base for purposes such as shape control, stabilized dispersion, for example of the pigment, and spreadability (smoothness) suitable for application to hair. The hair cosmetic according to the present invention can be made with known and commonly used oily bases.

The oily base is preferably a wax, which is solid at room temperature (25°C), particularly when used in a semi-solid hair cosmetic, such as a gel or cream. Examples of such waxes include vegetable waxes, such as candelilla wax, carnauba wax, rice bran wax, Japan wax, and sunflower wax, animal waxes, such as beeswax, mineral waxes such as ozokerite, ceresin, and microcrystalline wax, petroleum waxes, such as solid paraffin, and synthetic waxes, such as silicone wax and synthetic beeswax. A resin for film formation, such as (alkyl acrylate/diacetone acrylamide) copolymer AMP or a (methacryloyloxyethyl carboxybetaine/alkyl methacrylate) copolymer, may be contained. One of such waxes may be used alone, or two or more may be used in combination.

The amount of such a wax when it is used is, for example, 30% by mass or more, preferably 50% by mass or more, of the entire oily base. The amount of the wax when it is contained is, for example, from 20% to 90% by mass, preferably from 40% to 80% by mass, of the total amount (on a solids basis) of the hair cosmetic.

Besides a wax as described above, the hair cosmetic according to the present invention may be made with a liquid oily ingredient as an oily base. The liquid oily ingredient contained is preferably a polyhydric alcohol for preparation stability and dye solubility reasons. Examples of polyhydric alcohols include ethylene glycol, propylene glycol, dipropylene glycol, polyethylene glycol, 1,3-butylene glycol, glycerin, concentrated glycerin, diglycerin, triglycerin, glucose, maltose, maltitol, sucrose, mannitol, sorbitol, 1,2-pentanediol, 1,2-hexanediol, and 1,2-octanediol. The amount of the polyhydric alcohol when it is contained is, for example, from 0.5% to 10.0% by mass, preferably from 1.0% to 8.0% by mass, of the total amount of the hair cosmetic.

The hair cosmetic according to the present invention preferably contains a higher alcohol as a liquid oily ingredient for stability over time. Examples of higher alcohols include cetyl alcohol, stearyl alcohol, isostearyl alcohol, 2-octyldodecanol, and oleyl alcohol. The amount of the higher alcohol when it is contained is, for example, from 1.0% to 10.0% by mass, preferably from 2.0% to 9.0% by mass, of the total amount of the hair cosmetic.

The hair cosmetic according to the present invention, furthermore, preferably contains a hydrocarbon oil as a liquid oily ingredient for smoothness on the hair while being applied. Examples of the hydrocarbon oil include α-olefin oligomers, light isoparaffin, light liquid isoparaffin, squalane, synthetic squalane, vegetable squalane, liquid isoparaffin, and liquid paraffin. The amount of the hydrocarbon oil when it is contained is, for example, from 0.5% to 10.0% by mass, preferably from 1.0% to 8.0% by mass, of the total amount of the hair cosmetic.

In addition to the foregoing, the hair cosmetic may contain, as an oily base, a substance such as a solid oil, semi-solid oil, liquid oil, volatile oil, hydrocarbon, food or industrial oil, hydrogenated oil, ester oil, or fatty acid of animal oil, vegetable oil, synthetic oil, or other origin commonly used in cosmetics and hairdressing materials. Specifically, the hair cosmetic may contain, for example, hydrocarbons, such as liquid paraffin, squalane, polyisobutylene, and polybutene, food or industrial oils, such as olive oil, castor oil, and macadamia nut oil, esters, such as cetyl isooctanoate, isopropyl myristate, isopropyl palmitate, octyldodecyl myristate, and polyglyceryl diisostearate, and fatty acids, such as stearic acid, lauric acid, myristic acid, behenic acid, isostearic acid, and oleic acid.

### Nonionic Surfactant

The hair cosmetic according to the present invention, furthermore, preferably contains a nonionic surfactant for stability over time. Examples of the nonionic surfactant include polyoxyethylene fatty acids, glycerol esters of fatty acids, polyoxyethylene glycerol esters of fatty acids, polyglycerol esters of fatty acids, sorbitan esters of fatty acids, polyoxyethylene sorbitan esters of fatty acids, sucrose esters of fatty acids, polyoxyethylene alkyl ethers, polyoxyethylene alkyl phenyl ethers, polyglycerol alkyl ethers, dimethicone polyol, polyoxyethylene (hydrogenated) castor oil, and alkylol amides of fatty acids. The amount of the nonionic surfactant when it is contained is, for example, from 0.5% to 10.0% by mass, preferably from 1.0% to 8.0% by mass, of the total amount of the hair cosmetic.

### Aqueous Ingredient

The hair cosmetic according to the present invention may be of oil-based type, in which the base component is an oily ingredient, or may be of an emulsion type in which an aqueous ingredient has been blended into an oily ingredient (O/W emulsion type) or of W/O type, in which an oily ingredient has been blended into an aqueous ingredient. The amount of the aqueous ingredient when it is blended is, for example, from 30.0% to 90.0% by mass, preferably from 40.0% to 80.0% by mass.

The hair cosmetic according to the present invention may contain a water-soluble ingredient as an aqueous ingredient other than water. Examples of such aqueous ingredients include glycols, such as propylene glycol, 1,3-butylene glycol, dipropylene glycol, and polyethylene glycol, glycerols, such as glycerin, diglycerin, and polyglycerin, sugar alcohols, such as sorbitol, maltitol, and glucose, and lower alcohols, such as ethanol. The aqueous ingredient, furthermore, may contain a plant extract, for example from aloe vera, witch hazel, hamamelis, cucumbers, lemons, lavender, or rose.

### Other Ingredients

The hair cosmetic according to the present invention may contain, as ingredients other than the foregoing, commonly used additives (e.g., antioxidants, active cosmetic ingredients, preservatives, pH-adjusting agents, coolants, ultraviolet absorbers, dissolution aids, antibacterial agents, moisturizers, fragrances, amino acids, vitamins, medicinal plant extracts, and chelating agents). The amounts of these additives are, for example, approximately from 0.1% to 10 % by mass each of the total amount (on a solids basis) of the hair cosmetic.

Examples of antioxidants include α-tocopherol and ascorbic acid. Examples of active cosmetic ingredients include vitamins, anti-inflammatory agents, and herbal medicines. Examples of preservatives include paraoxybenzoates and phenoxyethanol. Examples of pH-adjusting agents include lactic acid, citric acid, glycolic acid, succinic acid, tartaric acid, and malic acid or their salts and potassium carbonate, sodium hydrogen carbonate, and ammonium hydrogen carbonate. Examples of coolants include L-menthol and camphor.

Examples of ultraviolet absorbers include 2-hydroxy-4-methoxybenzophenone, 2,4,6-trianilino-p-(carbo-2'-ethylhexyl-1'-oxy)-1,3,5-triazine, 2-ethylhexyl salicylate, ethyl paradihydroxypropylbenzoate, 2-ethylhexyl paramethoxycinnamate, 4-tert-4'-methoxydibenzoylmethane, hexyl 2-[4-(diethylamino)-2-hydroxybenzoyl]benzoate, 2-ethylhexyl dimethoxybenzylidenedioxoimidazolidinepropionate, 2,2'-methylenebis[6-(2H-benzotriazol-2-yl)-4-(1,1,3,3-tetramethylbutyl)phenol], (1,3,5)-triazine-2,4-bis[{4-(2-ethylhexyloxy)-2-hydroxy}phenyl]-6-(4-methoxyphenyl)-1,3,5-triazine, dimethycodiethyl benzalmalonate, and 2-hydroxy-4-methoxybenzophenone-5-sulfonic acid and its sodium salt.

Examples of dissolution aids include ethanol, glycerin, and sorbitol. Examples of preservatives and antibacterial agents include paraoxybenzoates, benzoic acid, sodium benzoate, sorbic acid, potassium sorbate, phenoxyethanol, salicylic acid, carbolic acid, sorbic acid, parachlorometacresol, hexachlorophene, benzalkonium chlorides, chlorhexidine chloride, trichlorocarbanilide, dehydroacetates, photosensitizing dyes, isopropylmethylphenol, pentylene glycol, hexylene glycol, caprylyl glycol, methylparaben, and anisic acid. Examples of antioxidants include tocopherol, butylhydroxyanisole, and dibutylhydroxytoluene.

When the hair cosmetic according to the present invention is a hair coloring agent (bleaching agent) that meets the standards for quasi-drugs, such as a hair dye or hair bleach, it may be of two-component mixture type, in which first and second agents are mixed together. The first agent typically contains ingredients such as an oxidative dye (dye that develops color through oxidation reaction), a coupler (toner), a direct dye, a pH-adjusting agent, a cream base (food or industrial oil + surfactant), a conditioning agent (humectant), a stabilizer, fragrances, and solvents. The second agent, on the other hand, contains ingredients such as an oxidizing agent, such as hydrogen peroxide solution, a pH-adjusting agent, an emulsion base, a stabilizer, fragrances, and solvents.

When the hair cosmetic according to the present invention is a hair spray or hair foam (mousse), a gas required for ejection, such as LPG, may optionally be contained in a mixture or dispersion containing the water-insoluble pigment composite according to the present invention and ingredients as described above.

### Method for Producing the Hair Cosmetic

The hair cosmetic according to the present invention can be produced by known and commonly used methods by preparing a mixture or dispersion containing the water-insoluble pigment composite according to the present invention and ingredients as described above. The equipment for production can be of any type as long as it is a disperser or emulsifier like a commonly used dispersion machine. The hair cosmetic according to the present invention can be implemented in various forms, such as an emulsion, cream, mousse, and a solid. The hair cosmetic, furthermore, can be packaged in different containers, such as a dispenser, a pump, a tube, a jar, and an aerosol can, according to the form of the preparation.

### EXAMPLES

The present invention will now be described in further detail with examples. The scope of the present invention, however, is not limited to these examples.

### EXAMPLE 1

To a 3-L beaker, 30.0 g of organophilized bentonite (MOISTNITE-WO, manufactured by Kunimine Industries Co., Ltd.) and 100 g of ethanol (Cica extra pure, manufactured by Kanto Chemical Co., Inc.) were added. Then 1000 g of deionized water was added, and the resulting mixture was stirred for 5 minutes at room temperature with glass stirring blades connected to a Three-One motor. In this manner, an aqueous dispersion of organophilized bentonite was prepared. To a 500-mL beaker 3.0 g of the annatto extract (manufactured by Kanto Chemical Co., Inc.), 300 mL of deionized water, and a stirrer were added, and the resulting mixture was stirred for 15 minutes at room temperature using a magnetic stirrer. In this manner, an annatto extract solution was prepared. The annatto extract solution was added to the aqueous dispersion of organophilized bentonite, and the resulting mixture was stirred for 15 minutes at room temperature. Then dilute hydrochloric acid prepared by diluting hydrochloric acid (Cica extra pure, manufactured by Kanto Chemical Co., Inc.) tenfold with deionized water was slowly added dropwise with a dropper to make the pH 4.0, and the resulting mixture was stirred for 2 hours at room temperature. One drop of the orange slurry was put on a sheet of filter paper, and it was observed that the spot displayed an orange color in a circular shape, and then a colorless and transparent liquid spread concentrically, indicating that the annatto extract was insolubilized. This slurry was filtered using a Nutsche filter, and the resulting solid was dried in a vacuum dryer (740 mmHg) at 30°C for 14 hours and then ground using a juicer, giving 29.1 g of powder (1). The ratio between the amount of organophilized bentonite and that of the annatto extract in powder (1) is 10:1 as a ratio of the amounts initially added. The resulting powder exhibited an orange color similar to that of the annatto extract.

### Preparation of Hair Dye Cream (1)

Into a 50-mL beaker 11.4 g of distilled water, 1.12 g of propylene glycol (manufactured by Koyo Fine Chemical Corporation), 0.28 g of polyquaternium-37-dicaprylyl carbonate-lauryl glucoside (manufactured by BASF Japan Ltd.), and 0.016 g of EDTA-2Na (manufactured by BASF Japan Ltd.) were measured out, and the ingredients were mixed together using a stirrer until uniform. Then 3.2 g of powder (1) (0.29 g as a pigment component) was added, and the ingredients were mixed together using a spatula until uniform. Then 0.048 g of methylparaben (manufactured by Maruzen Chemicals Co., Ltd.) and 0.032 g of propylparaben (manufactured by Maruzen Chemicals Co., Ltd.) were added, and the ingredients were mixed together using a spatula until uniform. In this manner, hair dye cream (1) was prepared.

### Evaluation of Hair Dye Cream (1)

### Color Strength Evaluations

### Color Strength When Applied to Human Blond Hair

Hair dye cream (1) was spread by hand on human blond hair, and color strength was evaluated visually. The human blond hair on which hair dye cream (1) was spread displayed a vivid orange color.

### Color Strength When Applied to Human Black Hair

Hair dye cream (1) was spread by hand on human black hair, and color strength was evaluated visually. The human black hair on which hair dye cream (1) was spread displayed a vivid orange color.

### Color Strength When Applied to Artificial Skin

Hair dye cream (1) was spread on artificial skin in a circular motion with a finger, and color strength was evaluated visually. The artificial skin on which hair dye cream (1) was spread displayed a vivid orange color. Evaluation of Water Resistance

Hair dye cream (1) was spread on a sheet of filter paper in a circular motion with a finger and allowed to dry for approximately 10 minutes at room temperature. Then 1 mL of tap water was dropped onto the center of the colored area with a dropper. It was observed that the spot continued displaying an orange color as before the dropping of water, and a colorless and transparent solution spread concentrically from the colored area. The area displaying an orange color represented the pigment component of hair dye cream (1); hair dye cream (1) did not bleed into water. Evaluation of Pigmentation

Hair dye cream (1) was applied evenly to human blond hair and allowed to dry for approximately 10 minutes at room temperature. The human blond hair was washed manually using a volume of shampoo (LUX super rich shine DAMAGE REPAIR shampoo for moist repair, manufactured by Unilever Japan K.K.) equivalent to a half-push (the amount dispensed when the pump is pushed halfway down), and the shampoo was thoroughly rinsed off with tap water at room temperature. Afterwards, a volume of conditioner (LUX super rich shine DAMAGE REPAIR conditioner for moist repair, manufactured by Unilever Japan K.K.) equivalent to a half-push was applied and then rinsed off with tap water at room temperature. After drying with warm air from a hair dryer for approximately 2 minutes, the washed human blond hair was inspected for any residual color. The hair displayed a golden color, indicating that hair dye cream (1) causes no pigmentation.

### EXAMPLE 2

In a 2-L glass beaker, 25.5 g of aluminum chloride(III) hexahydrate (manufactured by Kanto Chemical Co., Inc.) was dissolved in 858 mL of deionized water at room temperature (20°C). A 5% aqueous solution of sodium hydroxide prepared from a 48% aqueous solution of sodium hydroxide (manufactured by Kanto Chemical Co., Inc.) was added to make the pH 4.0, giving a slurry of aluminum hydroxide. Then 49.7 g of LINABLUE G1 (manufactured by DIC LIFETEC Co., Ltd., 55% trehalose, 40% spirulina extract, and 5% trisodium citrate) was added in powder form, and the resulting mixture was stirred for 30 minutes at room temperature (20°C). Then the pH was adjusted from 4.0 to 7.0 by adding a 5% aqueous solution of sodium hydroxide. This caused aluminum hydroxide to precipitate, yielding a blue slurry. After 1 hour of stirring, one drop of the blue slurry was put on a sheet of filter paper. It was observed that the spot displayed a blue color in a circular shape, and then a colorless and transparent liquid spread concentrically, indicating that phycocyanin was insolubilized. The blue slurry was filtered through a sheet of filter paper using a Nutsche filter, and the residue was washed with 2000 g of deionized water, giving a blue wet cake. The wet cake was dried in a vacuum dryer (740 mmHg) for 12 hours at room temperature (20°C), then dried in a drying oven at 50°C for 5 hours, and then ground using a juicer, giving 28.1 g of powder (2), a powder of aluminum hydroxide coated with phycocyanin. The ratio between the amount of aluminum hydroxide and that of the phycocyanin pigment in powder (2), determined by calculating the aluminum hydroxide content from the amount of it initially added and subtracting it from the actual weight of powder (2), was aluminum hydroxide:phycocyanin = 29:71 as a ratio by mass. The resulting powder (2) exhibited a blue color similar to that of phycocyanin.

### Preparation of Hair Dye Cream (2)

Into a 50-mL beaker 11.4 g of distilled water, 1.12 g of propylene glycol (manufactured by Koyo Fine Chemical Corporation), 0.28 g of polyquaternium-37-dicaprylyl carbonate-lauryl glucoside (manufactured by BASF Japan Ltd.), and 0.016 g of EDTA-2Na (manufactured by BASF Japan Ltd.) were measured out, and the ingredients were mixed together using a stirrer until uniform. Then 3.2 g of powder (2) (2.26 g as a pigment component) was added, and the ingredients were mixed together using a spatula until uniform. Then 0.048 g of methylparaben (manufactured by Maruzen Chemicals Co., Ltd.) and 0.032 g of propylparaben (manufactured by Maruzen Chemicals Co., Ltd.) were added, and the ingredients were mixed together using a spatula until uniform. In this manner, hair dye cream (2) was prepared.

### Evaluation of Hair Dye Cream (2)

### Color Strength Evaluations

### Color Strength When Applied to Human Blond Hair

Hair dye cream (2) was spread by hand on human blond hair, and color strength was evaluated visually. The human blond hair on which hair dye cream (2) was spread displayed a vivid indigo color.

### Color Strength When Applied to Human Black Hair

Hair dye cream (2) was spread by hand on human black hair, and color strength was evaluated visually. The human black hair on which hair dye cream (2) was spread displayed a vivid indigo color.

### Color Strength When Applied to Artificial Skin

Hair dye cream (2) was spread on artificial skin in a circular motion with a finger, and color strength was evaluated visually. The artificial skin on which hair dye cream (2) was spread displayed a vivid indigo color. Evaluation of Water Resistance

Hair dye cream (2) was spread on a sheet of filter paper in a circular motion with a finger and allowed to dry for approximately 10 minutes at room temperature. Then 1 mL of tap water was dropped onto the center of the colored area with a dropper. It was observed that the spot continued displaying an indigo color as before the dropping of water, and a colorless and transparent solution spread concentrically from the colored area. The area displaying an indigo color represented the pigment component of hair dye cream (2); hair dye cream (2) did not bleed into water. Evaluation of Pigmentation

Hair dye cream (2) was applied evenly to human blond hair and allowed to dry for approximately 10 minutes at room temperature. The human blond hair was washed manually using a volume of shampoo (LUX super rich shine DAMAGE REPAIR shampoo for moist repair, manufactured by Unilever Japan K.K.) equivalent to a half-push, and the shampoo was thoroughly rinsed off with tap water at room temperature. Afterwards, a volume of conditioner (LUX super rich shine DAMAGE REPAIR conditioner for moist repair, manufactured by Unilever Japan K.K.) equivalent to a half-push was applied and then rinsed off with tap water at room temperature. After drying with warm air from a hair dryer for approximately 2 minutes, the washed human blond hair was inspected for any residual color. The hair displayed a golden color, indicating that hair dye cream (2) causes no pigmentation.

### EXAMPLE 3

To a 3-L beaker 20.0 g of organophilized bentonite (MOISTNITE-WO, manufactured by Kunimine Industries Co., Ltd.) and 100 g of ethanol (Cica extra pure, manufactured by Kanto Chemical Co., Inc.) were added, and the bentonite was allowed to wet sufficiently. Then 1000 g of deionized water was added, and the resulting mixture was stirred for 5 minutes at room temperature with glass stirring blades connected to a Three-One motor. In this manner, an aqueous dispersion of organophilized bentonite was prepared. To a 500-mL beaker 2.0 g of sodium copper chlorophyllin (FUJIFILM Wako Pure Chemical Corporation), 300 mL of deionized water, and a stirrer were added, and the resulting mixture was stirred for 15 minutes at room temperature using a magnetic stirrer. In this manner, a pigment solution was prepared. The pigment solution was added to the aqueous dispersion, and the resulting mixture was stirred for 15 minutes at room temperature. Then dilute hydrochloric acid prepared by diluting hydrochloric acid (Cica extra pure, manufactured by Kanto Chemical Co., Inc.) tenfold with deionized water was slowly added dropwise with a dropper to make the pH 4.0, and the resulting mixture was stirred for 2 hours at room temperature. One drop of the green slurry was put on a sheet of filter paper, and it was observed that the spot displayed a green color in a circular shape, and then a colorless and transparent liquid spread concentrically, indicating that the sodium copper chlorophyllin was insolubilized. This slurry was filtered using a Nutsche filter, the residue was washed with 2000 g of deionized water, and the resulting solid was dried in a vacuum dryer (740 mmHg) at 30°C for 14 hours and then ground using a juicer, giving 18.1 g of powder (3). The ratio between the amount of organophilized bentonite and that of sodium copper chlorophyllin in the resulting powder (3) is 10:1 as a ratio of the amounts initially added. The resulting powder exhibited a green color similar to that of sodium copper chlorophyllin.

### Preparation of Hair Dye Cream (3)

Into a 50-mL beaker 11.4 g of distilled water, 1.12 g of propylene glycol (manufactured by Koyo Fine Chemical Corporation), 0.28 g of polyquaternium-37-dicaprylyl carbonate-lauryl glucoside (manufactured by BASF Japan Ltd.), and 0.016 g of EDTA-2Na (manufactured by BASF Japan Ltd.) were measured out, and the ingredients were mixed together using a stirrer until uniform. Then 3.2 g of powder (3) (0.29 g as a pigment component) was added, and the ingredients were mixed together using a spatula until uniform. Then 0.048 g of methylparaben (manufactured by Maruzen Chemicals Co., Ltd.) and 0.032 g of propylparaben (manufactured by Maruzen Chemicals Co., Ltd.) were added, and the ingredients were mixed together using a spatula until uniform. In this manner, hair dye cream (3) was prepared.

### Evaluation of Hair Dye Cream (3)

### Color Strength Evaluations

### Color Strength When Applied to Human Blond Hair

Hair dye cream (3) was spread by hand on human blond hair, and color strength was evaluated visually. The human blond hair on which hair dye cream (3) was spread displayed a vivid green color.

### Color Strength When Applied to Human Black Hair

Hair dye cream (3) was spread by hand on human black hair, and color strength was evaluated visually. The human black hair on which hair dye cream (3) was spread displayed a vivid green color.

### Color Strength When Applied to Artificial Skin

Hair dye cream (3) was spread on artificial skin in a circular motion with a finger, and color strength was evaluated visually. The artificial skin on which hair dye cream (3) was spread displayed a vivid green color. Evaluation of Water Resistance

Hair dye cream (3) was spread on a sheet of filter paper in a circular motion with a finger and allowed to dry for approximately 10 minutes at room temperature. Then 1 mL of tap water was dropped onto the center of the colored area with a dropper. It was observed that the spot continued displaying a green color as before the dropping of water, and a colorless and transparent solution spread concentrically from the colored area. The area displaying a green color represented the pigment component of hair dye cream (3); the pigment component of hair dye cream (3) did not bleed into water.

### Evaluation of Pigmentation

Hair dye cream (3) was applied evenly to human blond hair and allowed to dry for approximately 10 minutes at room temperature. The human blond hair was washed manually using a volume of shampoo (LUX super rich shine DAMAGE REPAIR shampoo for moist repair, manufactured by Unilever Japan K.K.) equivalent to a half-push, and the shampoo was thoroughly rinsed off with tap water at room temperature. Afterwards, a volume of conditioner (LUX super rich shine DAMAGE REPAIR conditioner for moist repair, manufactured by Unilever Japan K.K.) equivalent to a half-push was applied and then rinsed off with tap water at room temperature. After drying with warm air from a hair dryer for approximately 2 minutes, the washed human blond hair was inspected for any residual color. The hair displayed a golden color, indicating that hair dye cream (3) causes no pigmentation.

### EXAMPLE 4

To a 3-L beaker 20.0 g of hydroxyapatite (manufactured by FUJIFILM Wako Pure Chemical Corporation) and 1000 g of deionized water were added, and the resulting mixture was stirred for 5 minutes at room temperature with glass stirring blades connected to a Three-One motor. In this manner, an aqueous dispersion of hydroxyapatite was prepared. To a 500-mL beaker 2.0 g of sodium copper chlorophyllin (FUJIFILM Wako Pure Chemical Corporation), 300 mL of deionized water, and a stirrer were added, and the resulting mixture was stirred for 15 minutes at room temperature using a magnetic stirrer. In this manner, a pigment solution was prepared. The pigment solution was added to the aqueous dispersion of hydroxyapatite, and the resulting mixture was stirred for 15 minutes at room temperature. Then dilute hydrochloric acid prepared by diluting hydrochloric acid (Cica extra pure, manufactured by Kanto Chemical Co., Inc.) tenfold with deionized water was slowly added dropwise with a dropper to make the pH 4.0, and the resulting mixture was stirred for 2 hours at room temperature. One drop of the green slurry was put on a sheet of filter paper, and it was observed that the spot displayed a green color in a circular shape, and then a colorless and transparent liquid spread concentrically, indicating that the sodium copper chlorophyllin was insolubilized. This slurry was filtered using a Nutsche filter, the residue was washed with 2000 g of deionized water, and the resulting solid was dried in a vacuum dryer (740 mmHg) at 30°C for 14 hours and then ground using a juicer, giving 20.2 g of powder (4). The ratio between the amount of hydroxyapatite and that of sodium copper chlorophyllin in powder (4) is 10:1 as a ratio of the amounts initially added. The resulting powder exhibited a green color similar to that of sodium copper chlorophyllin. Preparation of Hair Dye Cream (4)

Into a 50-mL beaker 11.4 g of distilled water, 1.12 g of propylene glycol (manufactured by Koyo Fine Chemical Corporation), 0.28 g of polyquaternium-37-dicaprylyl carbonate-lauryl glucoside (manufactured by BASF Japan Ltd.), and 0.016 g of EDTA-2Na (manufactured by BASF Japan Ltd.) were measured out, and the ingredients were mixed together using a stirrer until uniform. Then 3.2 g of powder (4) (0.29 g as a pigment component) was added, and the ingredients were mixed together using a spatula until uniform. Then 0.048 g of methylparaben (manufactured by Maruzen Chemicals Co., Ltd.) and 0.032 g of propylparaben (manufactured by Maruzen Chemicals Co., Ltd.) were added, and the ingredients were mixed together using a spatula until uniform. In this manner, hair dye cream (4) was prepared.

### Methods for the Evaluation of the Hair Dye

### Color Strength Evaluations

### Color Strength When Applied to Human Blond Hair

Hair dye cream (4) was spread by hand on human blond hair, and color strength was evaluated visually. The human blond hair on which hair dye cream (4) was spread displayed a green color.

### Color Strength When Applied to Human Black Hair

Hair dye cream (4) was spread by hand on human black hair, and color strength was evaluated visually. The human black hair on which hair dye cream (4) was spread displayed a green color.

### Color Strength When Applied to Artificial Skin

Hair dye cream (4) was spread on artificial skin in a circular motion with a finger, and color strength was evaluated visually. The artificial skin on which hair dye cream (4) was spread displayed a green color.

### Evaluation of Water Resistance

Hair dye cream (4) was spread on a sheet of filter paper in a circular motion with a finger and allowed to dry for approximately 10 minutes at room temperature. Then 1 mL of tap water was dropped onto the center of the colored area with a dropper. It was observed that the spot continued displaying a green color as before the dropping of water, and a colorless and transparent solution spread concentrically from the colored area. The area displaying a green color represented hair dye cream (4); hair dye cream (4) did not bleed into water.

### Evaluation of Pigmentation

Hair dye cream (4) was applied evenly to human blond hair and allowed to dry for approximately 10 minutes at room temperature. The human blond hair was washed manually using a volume of shampoo (LUX super rich shine DAMAGE REPAIR shampoo for moist repair, manufactured by Unilever Japan K.K.) equivalent to a half-push, and the shampoo was thoroughly rinsed off with tap water at room temperature. Afterwards, a volume of conditioner (LUX super rich shine DAMAGE REPAIR conditioner for moist repair, manufactured by Unilever Japan K.K.) equivalent to a half-push was applied and then rinsed off with tap water at room temperature. After drying with warm air from a hair dryer for approximately 2 minutes, the washed human blond hair was inspected for any residual color. The hair displayed a golden color, indicating that hair dye cream (4) causes no pigmentation.

### EXAMPLE 5

To a 3-L beaker 20.0 g of organophilized bentonite (MOISTNITE-WO, manufactured by Kunimine Industries Co., Ltd.) and 100 g of ethanol (Cica extra pure, manufactured by Kanto Chemical Co., Inc.) were added, and the bentonite was allowed to wet sufficiently. Then 1000 g of deionized water was added, and the resulting mixture was stirred for 5 minutes at room temperature with glass stirring blades connected to a Three-One motor. In this manner, an aqueous dispersion of organophilized bentonite was prepared. To a 500-mL beaker 1.18 g of Carthamus yellow (manufactured by Daiwa Dyestuff Mfg. Co., Ltd., 85% Carthamus yellow and 15% dextrin), 300 mL of deionized water, and a stirrer were added, and the resulting mixture was stirred for 15 minutes at room temperature using a magnetic stirrer. In this manner, a pigment solution was prepared. The pigment solution was added to the aqueous dispersion, and the resulting mixture was stirred for 15 minutes at room temperature. Then dilute hydrochloric acid prepared by diluting hydrochloric acid (Cica extra pure, manufactured by Kanto Chemical Co., Inc.) tenfold with deionized water was slowly added dropwise with a dropper to make the pH 4.0, and the resulting mixture was stirred for 2 hours at room temperature. One drop of the yellow slurry was put on a sheet of filter paper, and it was observed that the spot displayed a yellow color in a circular shape, and then a colorless and transparent liquid spread concentrically, indicating that the Carthamus yellow was insolubilized. This slurry was filtered using a Nutsche filter, the residue was washed with 2000 g of deionized water, and the resulting solid was dried in a vacuum dryer (740 mmHg) at 30°C for 14 hours and then ground using a juicer, giving 20.1 g of powder (5). The ratio between the amount of organophilized bentonite and that of Carthamus yellow in the resulting powder (5) is 20:1 as a ratio of the amounts initially added. The resulting powder exhibited a yellow color similar to that of Carthamus yellow.

### Preparation of Hair Dye Cream (5)

Into a 50-mL beaker 11.4 g of distilled water, 1.12 g of propylene glycol (manufactured by Koyo Fine Chemical Corporation), 0.28 g of polyquaternium-37-dicaprylyl carbonate-lauryl glucoside (manufactured by BASF Japan Ltd.), and 0.016 g of EDTA-2Na (manufactured by BASF Japan Ltd.) were measured out, and the ingredients were mixed together using a stirrer until uniform. Then 3.2 g of powder (5) (0.15 g as a pigment component) was added, and the ingredients were mixed together using a spatula until uniform. Then 0.048 g of methylparaben (manufactured by Maruzen Chemicals Co., Ltd.) and 0.032 g of propylparaben (manufactured by Maruzen Chemicals Co., Ltd.) were added, and the ingredients were mixed together using a spatula until uniform. In this manner, hair dye cream (5) was prepared.

### Methods for the Evaluation of the Hair Dye

### Color Strength Evaluations

### Color Strength When Applied to Human Blond Hair

Hair dye cream (5) was spread by hand on human blond hair, and color strength was evaluated visually. The human blond hair on which hair dye cream (5) was spread displayed a vivid yellow color.

### Color Strength When Applied to Human Black Hair

Hair dye cream (5) was spread by hand on human black hair, and color strength was evaluated visually. The human black hair on which hair dye cream (5) was spread displayed a vivid yellow color.

### Color Strength When Applied to Artificial Skin

Hair dye cream (5) was spread on artificial skin in a circular motion with a finger, and color strength was evaluated visually. The artificial skin on which hair dye cream (5) was spread displayed a vivid yellow color. Evaluation of Water Resistance

Hair dye cream (5) was spread on a sheet of filter paper in a circular motion with a finger and allowed to dry for approximately 10 minutes at room temperature. Then 1 mL of tap water was dropped onto the center of the colored area with a dropper. It was observed that the spot continued displaying a yellow color as before the dropping of water, and a colorless and transparent solution spread concentrically from the colored area. The area displaying a yellow color represented hair dye cream (5); hair dye cream (5) did not bleed into water.

### Evaluation of Pigmentation

Hair dye cream (5) was applied evenly to human blond hair and allowed to dry for approximately 10 minutes at room temperature. The human blond hair was washed manually using a volume of shampoo (LUX super rich shine DAMAGE REPAIR shampoo for moist repair, manufactured by Unilever Japan K.K.) equivalent to a half-push, and the shampoo was thoroughly rinsed off with tap water at room temperature. Afterwards, a volume of conditioner (LUX super rich shine DAMAGE REPAIR conditioner for moist repair, manufactured by Unilever Japan K.K.) equivalent to a half-push was applied and then rinsed off with tap water at room temperature. After drying with warm air from a hair dryer for approximately 2 minutes, the washed human blond hair was inspected for any residual color. The hair displayed a golden color, indicating that hair dye cream (5) causes no pigmentation.

### COMPARATIVE EXAMPLE 1

### Preparation of Hair Dye Cream (6)

Into a 50-mL beaker 14.0 g of distilled water, 1.38 g of propylene glycol (manufactured by Koyo Fine Chemical Corporation), 0.35 g of polyquaternium-37-dicaprylyl carbonate-lauryl glucoside (manufactured by BASF Japan Ltd.), and 0.02 g of EDTA-2Na (manufactured by BASF Japan Ltd.) were measured out, and the ingredients were mixed together using a stirrer until uniform. Then 0.29 g of annatto extract (manufactured by Kanto Chemical Co., Inc.) was added, and the ingredients were mixed together using a spatula until uniform. Then 0.048 g of methylparaben (manufactured by Maruzen Chemicals Co., Ltd.) and 0.032 g of propylparaben (manufactured by Maruzen Chemicals Co., Ltd.) were added, and the ingredients were mixed together using a spatula until uniform. In this manner, hair dye cream (6) was prepared.

### Methods for the Evaluation of the Hair Dye

### Color Strength Evaluations

### Color Strength When Applied to Human Blond Hair

Hair dye cream (6) was spread by hand on human blond hair, and color strength was evaluated visually. The human blond hair on which hair dye cream (6) was spread displayed a vivid orange color.

### Color Strength When Applied to Human Black Hair

Hair dye cream (6) was spread by hand on human black hair, and color strength was evaluated visually. The human black hair on which hair dye cream (6) was spread was black and did not display any color.

### Color Strength When Applied to Artificial Skin

Hair dye cream (6) was spread on artificial skin in a circular motion with a finger, and color strength was evaluated visually. The artificial skin on which hair dye cream (6) was spread displayed a vivid orange color. Evaluation of Water Resistance

Hair dye cream (6) was spread on a sheet of filter paper in a circular motion with a finger and allowed to dry for approximately 10 minutes at room temperature. Then 1 mL of tap water was dropped onto the center of the colored area with a dropper. It was observed that the spot continued displaying an orange color as before the dropping of water, and a solution colored orange spread concentrically from the colored area. The area displaying an orange color represented hair dye cream (6), and the orange solution that concentrically spread was tap water with the annatto extract dissolved in it; hair dye cream (6) bled into water. Evaluation of Pigmentation

Hair dye cream (6) was applied evenly to human blond hair and allowed to dry for approximately 10 minutes at room temperature. The human blond hair was washed manually using a volume of shampoo (LUX super rich shine DAMAGE REPAIR shampoo for moist repair, manufactured by Unilever Japan K.K.) equivalent to a half-push, and the shampoo was thoroughly rinsed off with tap water at room temperature. Afterwards, a volume of conditioner (LUX super rich shine DAMAGE REPAIR conditioner for moist repair, manufactured by Unilever Japan K.K.) equivalent to a half-push was applied and then rinsed off with tap water at room temperature. After drying with warm air from a hair dryer for approximately 3 minutes, the washed human blond hair was inspected for any residual color. The human blond hair displayed an orange color; hair dye cream (6) caused pigmentation.

Results to this point are summarized in Table 1 below.

**Table 1**

| Hair cosmetic | Colorant | Color strength on artificial skin | Water resistance | Color strength on black hair | Pigmentation |
|---|---|---|---|---|---|
| Example 1 | Powder (1) | ○ | ○ | ○ | ○ |
| Comparative Example 1 | Annatto extract (dye) | ○ | × | × | × |

As shown in Table 1, hair dye cream (1), made with powder (1), was superior in terms of water resistance, color strength on black hair, and pigmentation.

### COMPARATIVE EXAMPLE 2

### Preparation of Hair Dye Cream (7)

Into a 50-mL beaker 9.22 g of distilled water, 0.92 g of propylene glycol (manufactured by Koyo Fine Chemical Corporation), 0.23 g of polyquaternium-37-dicaprylyl carbonate-lauryl glucoside (manufactured by BASF Japan Ltd.), and 0.01 g of EDTA-2Na (manufactured by BASF Japan Ltd.) were measured out, and the ingredients were mixed together using a stirrer until uniform. Then 5.65 g of LINABLUE G1 (manufactured by DIC LIFETEC Co., Ltd., 55% trehalose, 40% phycocyanin pigment, and 5% trisodium citrate) (2.26 g as a pigment component) was added, and the ingredients were mixed together using a spatula until uniform. Then 0.048 g of methylparaben (manufactured by Maruzen Chemicals Co., Ltd.) and 0.032 g of propylparaben (manufactured by Maruzen Chemicals Co., Ltd.) were added, and the ingredients were mixed together using a spatula until uniform. In this manner, hair dye cream (7) was prepared.

### Methods for the Evaluation of the Hair Dye

### Color Strength Evaluations

### Color Strength When Applied to Human Blond Hair

Hair dye cream (7) was spread by hand on human blond hair, and color strength was evaluated visually. The human blond hair on which hair dye cream (7) was spread displayed a vivid indigo color.

### Color Strength When Applied to Human Black Hair

Hair dye cream (7) was spread by hand on human black hair, and color strength was evaluated visually. The human black hair on which hair dye cream (7) was spread was black and did not display any color.

### Color Strength When Applied to Artificial Skin

Hair dye cream (7) was spread on artificial skin in a circular motion with a finger, and color strength was evaluated visually. The artificial skin on which hair dye cream (7) was spread displayed a vivid indigo color. Evaluation of Water Resistance

Hair dye cream (7) was spread on a sheet of filter paper in a circular motion with a finger and allowed to dry for approximately 10 minutes at room temperature. Then 1 mL of tap water was dropped onto the center of the colored area with a dropper. It was observed that the spot continued displaying an indigo color as before the dropping of water, and a solution colored indigo spread concentrically from the colored area. The area displaying an indigo color represented hair dye cream (7), and the indigo solution that concentrically spread was tap water with the phycocyanin pigment dissolved in it; hair dye cream (7) bled into water. Evaluation of Pigmentation

Hair dye cream (7) was applied evenly to human blond hair and allowed to dry for approximately 10 minutes at room temperature. The human blond hair was washed manually using a volume of shampoo (LUX super rich shine DAMAGE REPAIR shampoo for moist repair, manufactured by Unilever Japan K.K.) equivalent to a half-push, and the shampoo was thoroughly rinsed off with tap water at room temperature. Afterwards, a volume of conditioner (LUX super rich shine DAMAGE REPAIR conditioner for moist repair, manufactured by Unilever Japan K.K.) equivalent to a half-push was applied and then rinsed off with tap water at room temperature. After drying with warm air from a hair dryer for approximately 3 minutes, the washed human blond hair was inspected for any residual color. The hair displayed a golden color, indicating that hair dye cream (7) causes no pigmentation.

Results to this point are summarized in Table 2 below.

**Table 2**

| Hair cosmetic | Colorant | Color strength on artificial skin | Water resistance | Color strength on black hair | Pigmentation |
|---|---|---|---|---|---|
| Example 2 | Powder 2 | ○ | ○ | ○ | ○ |
| Comparative Example 2 | Phycocyanin pigment (dye) | ○ | × | × | ○ |

As shown in Table 2, hair dye cream (2), made with powder (2), was superior in terms of water resistance and color strength on black hair.

### COMPARATIVE EXAMPLE 3

### Preparation of Hair Dye Cream (8)

Into a 50-mL beaker 14.0 g of distilled water, 1.38 g of propylene glycol (manufactured by Koyo Fine Chemical Corporation), 0.35 g of polyquaternium-37-dicaprylyl carbonate-lauryl glucoside (manufactured by BASF Japan Ltd.), and 0.02 g of EDTA-2Na (manufactured by BASF Japan Ltd.) were measured out, and the ingredients were mixed together using a stirrer until uniform. Then 0.29 g of sodium copper chlorophyllin (FUJIFILM Wako Pure Chemical Corporation) was added, and the ingredients were mixed together using a spatula until uniform. Then 0.048 g of methylparaben (manufactured by Maruzen Chemicals Co., Ltd.) and 0.032 g of propylparaben (manufactured by Maruzen Chemicals Co., Ltd.) were added, and the ingredients were mixed together using a spatula until uniform. In this manner, hair dye cream (8) was prepared.

### Methods for the Evaluation of the Hair Dye

### Color Strength Evaluations

### Color Strength When Applied to Human Blond Hair

Hair dye cream (8) was spread by hand on human blond hair, and color strength was evaluated visually. The human blond hair on which hair dye cream (8) was spread displayed a deep green color.

### Color Strength When Applied to Human Black Hair

Hair dye cream (8) was spread by hand on human black hair, and color strength was evaluated visually. The human black hair on which hair dye cream (8) was spread was black and did not display any color.

### Color Strength When Applied to Artificial Skin

Hair dye cream (8) was spread on artificial skin in a circular motion with a finger, and color strength was evaluated visually. The artificial skin on which hair dye cream (8) was spread displayed a deep green color. Evaluation of Water Resistance

Hair dye cream (8) was spread on a sheet of filter paper in a circular motion with a finger and allowed to dry for approximately 10 minutes at room temperature. Then 1 mL of tap water was dropped onto the center of the colored area with a dropper. It was observed that the spot continued displaying a deep green color as before the dropping of water, and a colorless and transparent solution spread concentrically from the colored area. The area displaying a deep green color represented hair dye cream (8), and the colorless and transparent solution that concentrically spread was tap water; hair dye cream (8) did not bleed into water.

### Evaluation of Pigmentation

Hair dye cream (8) was applied evenly to human blond hair and allowed to dry for approximately 10 minutes at room temperature. The human blond hair was washed manually using a volume of shampoo (LUX super rich shine DAMAGE REPAIR shampoo for moist repair, manufactured by Unilever Japan K.K.) equivalent to a half-push, and the shampoo was thoroughly rinsed off with tap water at room temperature. Afterwards, a volume of conditioner (LUX super rich shine DAMAGE REPAIR conditioner for moist repair, manufactured by Unilever Japan K.K.) equivalent to a half-push was applied and then rinsed off with tap water at room temperature. After drying with warm air from a hair dryer for approximately 3 minutes, the washed human blond hair was inspected for any residual color. The human blond hair displayed a light green color; hair dye cream (8) caused pigmentation.

Results to this point are summarized in Table 3 below.

**Table 3**

| Hair cosmetic | Colorant | Color strength on artificial skin | Water resistance | Color strength on black hair | Pigmentation |
|---|---|---|---|---|---|
| Example 3 | Powder (3) | ○ | ○ | ○ | ○ |
| Example 4 | Powder (4) | ○ | ○ | ○ | ○ |
| Comparative Example 3 | Sodium copper chlorophyllin (dye) | ○ | ○ | × | × |

As shown in Table 3, hair dye creams (3) and (4), made with powders (3) and (4), respectively, were superior in terms of color strength on black hair and pigmentation.

### COMPARATIVE EXAMPLE 4

### Preparation of Hair Dye Cream (9)

Into a 50-mL beaker 14.1 g of distilled water, 1.38 g of propylene glycol (manufactured by Koyo Fine Chemical Corporation), 0.35 g of polyquaternium-37-dicaprylyl carbonate-lauryl glucoside (manufactured by BASF Japan Ltd.), and 0.02 g of EDTA-2Na (manufactured by BASF Japan Ltd.) were measured out, and the ingredients were mixed together using a stirrer until uniform. Then 0.18 g of Safflower Y1500 (manufactured by Daiwa Dyestuff Mfg. Co., Ltd., 85% Carthamus yellow and 15% dextrin) (0.15 g as a pigment component was added, and the ingredients were mixed together using a spatula until uniform. Then 0.048 g of methylparaben (manufactured by Maruzen Chemicals Co., Ltd.) and 0.032 g of propylparaben (manufactured by Maruzen Chemicals Co., Ltd.) were added, and the ingredients were mixed together using a spatula until uniform. In this manner, hair dye cream (9) was prepared.

### Methods for the Evaluation of the Hair Dye

### Color Strength Evaluations

### Color Strength When Applied to Human Blond Hair

Hair dye cream (9) was spread by hand on human blond hair, and color strength was evaluated visually. The human blond hair on which hair dye cream (9) was spread displayed a vivid yellow color.

### Color Strength When Applied to Human Black Hair

Hair dye cream (9) was spread by hand on human black hair, and color strength was evaluated visually. The human black hair on which hair dye cream (9) was spread was black and did not display any color.

### Color Strength When Applied to Artificial Skin

Hair dye cream (9) was spread on artificial skin in a circular motion with a finger, and color strength was evaluated visually. The artificial skin on which hair dye cream (9) was spread displayed a vivid yellow color. Evaluation of Water Resistance

Hair dye cream (9) was spread on a sheet of filter paper in a circular motion with a finger and allowed to dry for approximately 10 minutes at room temperature. Then 1 mL of tap water was dropped onto the center of the colored area with a dropper. It was observed that the spot continued displaying a vivid yellow color as before the dropping of water, and a solution colored yellow spread concentrically from the colored area. The area displaying a yellow color represented hair dye cream (9), and the yellow solution that concentrically spread was tap water; hair dye cream (9) bled into water.

### Evaluation of Pigmentation

Hair dye cream (9) was applied evenly to human blond hair and allowed to dry for approximately 10 minutes at room temperature. The human blond hair was washed manually using a volume of shampoo (LUX super rich shine DAMAGE REPAIR shampoo for moist repair, manufactured by Unilever Japan K.K.) equivalent to a half-push, and the shampoo was thoroughly rinsed off with tap water at room temperature. Afterwards, a volume of conditioner (LUX super rich shine DAMAGE REPAIR conditioner for moist repair, manufactured by Unilever Japan K.K.) equivalent to a half-push was applied and then rinsed off with tap water at room temperature. After drying with warm air from a hair dryer for approximately 3 minutes, the washed human blond hair was inspected for any residual color. The human blond hair displayed a yellow color; hair dye cream (9) caused pigmentation.

Results to this point are summarized in Table 4 below.

**Table 4**

| Hair cosmetic | Colorant | Color strength on artificial skin | Water resistance | Color strength on black hair | Pigmentation |
|---|---|---|---|---|---|
| Example 5 | Powder (5) | ○ | ○ | ○ | ○ |
| Comparative Example 4 | Carthamus yellow (dye) | ○ | × | × | × |

As shown in Table 4, hair dye cream (5), made with powder (5), was superior in terms of water resistance, color strength on black hair, and pigmentation.

## Claims

1. A hair cosmetic comprising a water-insoluble pigment composite including a complex of at least one natural pigment and at least one substrate.

2. The hair according to Claim 1, wherein a ratio between an amount of the natural pigment and an amount of the substrate in the water-insoluble pigment composite is the natural pigment:the substrate = 0.1:99.9 to 90:10 as a ratio by mass.

3. The hair cosmetic according to Claim 1 or 2,
wherein the substrate in the water-insoluble pigment composite is at least one selected from a metal oxide, a metal hydroxide, a clay mineral, hydroxyapatite, or an organic polysaccharide.

4. The hair cosmetic according to Claim 1 or 2,
wherein the natural pigment in the water-insoluble pigment composite is at least one selected from a flavonoid pigment, a carotenoid pigment, a porphyrin pigment, or a chromoprotein.

5. The hair cosmetic according to Claim 1 or 2,
wherein the natural pigment in the water-insoluble pigment composite is at least one selected from annatto extract, chlorophyll pigment, Carthamus yellow, or phycocyanin pigment.
